# EUROPEAN PATENT SPECIFICATION

(11) **EP 1 189 595 B1**
(45) Date of publication and mention of the grant of the patent: **14.05.2003**
(21) Application number: 00945849.8
(22) Date of filing: 29.06.2000
(51) Int. Cl.: A61K 9/06, A61P 31/04, A61P 31/10, A61P 29/00, A61P 9/10

(54) **OPHTHALMIC COMPOSITIONS IN FORM OF AQUEOUS GELS**
OPHTHALMISCHE ZUSAMMENSETZUNGEN IN FORM WÄSSRIGER GELE
COMPOSITIONS OPHTHALMIQUES SOUS FORME DE GELS AQUEUX

(30) Priority: 01.07.1999 IT MI991453
(43) Date of publication of application: 27.03.2002
(73) Proprietor: Farmila Farmaceutici Milano S.p.A., 20019 Settimo Milanese (IT)
(72) Inventor: SANSO', Marco, I-20019 Settimo Milanese (IT); SEBBEN, Renato, I-20019 Settimo Milanese (IT)
(74) Representative: Minoja, Fabrizio, Dr.
(86) International application number: EP0006062
(87) International publication number: WO01001959

(56) References cited:
- FR-A- 2 462 160
- US-A- 3 947 573
- US-A- 4 409 205
- US-A- 5 188 826
- US-A- 5 676 949
- US-A- 5 800 807

## Description

The present invention relates to ophthalmic compositions in form of aqueous gels.

The pharmacological therapy of ocular pathologies by topical administration may appear as a simple problem if many peculiar elements of the ocular structure, which make it of pharmaceutically difficult treatment, are not considered.

Not considering the pathology and, therefore, the adequate active principle, the mostly influencing variables to optimise this kind of therapy are the following:
- physiological/anatomical aspects; the administered quantity of active principle in the form of homogeneous aqueous solution or suspension (conventional eye drops) quickly leaves the interested area either by blinking and/or lacrimal drainage.
   Just a small percentage of the administered preparation is retained in the corneal region, and can develop, after absorption, a pharmacological action.
- "barrier" effect to absorption, induced by different tissues/components (cornea, conjunctiva, tear film etc.) influencing the active principle distribution in the different compartments of the anterior section of the eye.

In the last years, to solve the above mentioned problems, many strategies have been carried out , based on:
1) the chemical structure of the active principle (more addressed to hydrophilic/lipophilic ratio), therefore trying to increase absorption - in the cornea, conjunctiva etc. - of the slight residual quantity, after blinking;
2) the weight increase of the active principle in the formulations, with consequent higher risk of adverse effects;
3) the increase of the daily posology (up to 6/8 daily administrations) with reduction of patient compliance;
4) the change of the vehicle used in the pharmaceutical preparations.

As far as the latter point is concerned, the rational of different approaches, made up to now, consists in the hypothesis that an increase of residence time in the ocular milieu (cornea, conjunctiva) of the blinking-resistant residual dose should lead to an increase of therapeutical efficacy, due to high potential availability of the active principle absorption.

To reach this target different strategies have been adopted: i) use of solid devices with slow releasing effects to be placed in the cul-de-sac (scarcely accepted by the patient); ii) use of formulations characterised by a more or less high viscosity (mostly used approach).

Disregarding the use of eye ointments based on fat vehicles, whose limited compliance is well-known , the approach to viscous formulations can be divided into: i) use of preformed aqueous vehicles of appropriate viscosity, to be .administered as such; ii) use of suitable solutions, gelling in contact with ocular surface (for example by variation of temperature, pH or ionic strength).

US3,920,810 (18/11/1975), to Burton Parson & Co., claims low viscosity ophthalmic solutions, using polyacrylamide as first agent and, optionally, polyethylene glycol as wetting and plasticizer agent.

These solutions can be used as artificial tears or as cleaning and hydrating liquids for contact lenses, or as vehicles for chemically alkaline or neutral active substances. In this latter case, the following additives are suggested but, are not considered essential: - water-soluble cellulose derivatives, like hydroxymethylcellulose as buffer agents and viscosity stabilising agents; polyvinylpirrolidone, as "detoxicant" of lacrimal film and spreading agent; biocides etc.

The viscosity of these solutions ranges between 10 and 200 cps, while the high residence time, compared with that of conventional eye drops, seems to be triplicated (even quadruplicated); the comparison between a pilocarpine preparation and one in the form of traditional eye drops leads to similar therapeutic results, with a slightly reduction of the dosage (3 adm./day vs 4 adm./day).

FR76358076 (Alcon Laboratories) discloses solutions used as artificial tears, for dry eye syndrome treatment; these solutions show low viscosity and contain polysaccharides such as dextrans or arabinogalactans and polyethylene glycols.

EP-B-227494 (02/10/1986, Laboratories Merk, Sharp & Dohme-Chibret) discloses polysaccharide liquid preparations which, under the effect of a ionic strength variation (ocular milieu salinity), undergo transition to high or low viscosity.

The used polysaccharide is a heteropolysaccharide produced by the Pseudomonas Elodea bacterium, known as gellan gum or its modifications.

The formulations are useful in the treatment of dry eye syndrome or as drug delivering systems.

Said formulations show a residence time about twice that obtained by a traditional aqueous preparation.

The comparison between two compositions, with the same concentration (0.25%) of timolol (a non-gelling commercial one and the other containing a vehicle described in EP-B-227494), shows that on checking times (30;60;120;180 minutes) the active principle concentration of the commercial preparation in the rabbit lacrimal fluid is 3 (30;60;120 minutes) to two times (180 min.) versus the disclosed composition.

A drawback could consists in the fact that the optimal active substances should be soluble in water, while the non soluble substances have to be used in suspensions or in emulsions.

WO 91/19481 (11/06/91 Allergan Inc.) discloses solutions reversibly gelling upon simultaneous variations of, at least, two physical parameters, such as temperature, pH or ionic strength.

These solutions, suitable for topical or sistemic formulations, consist of a methylcellulose and polyacrylic acid mixture in the presence or in the absence of inorganic salts.

These drug vehicles can carry active substances in solutions or in dispersion. The final, local values of lacrimal fluid viscosity, formed after gelling, should be very high, considering that initial values ranged from 3000 cP to 16,000 cP and more, even if they are defined easily flowing in drops.

The residence time and the distribution have been studied by the fluoresceine test or FITC-dextran test, with the following conclusions:
i) formation of a gelatinous deposit in the lower part of the cul-de-sac,
ii) formation of a regular-looking, thin film on the conjunctival-corneal region.

These results show that an important part of the composition remains in an ocular site of low absorption capacity, where, moreover, the elimination by lacrimal drainage is easier (the residence time in the eye has been evaluated of about 3 hours, whereas the one of the film would be twice longer).

WO 93/17664 (Alcon lab.) discloses ophthalmic preparations useful *per se* as drugs for the dry eye syndrome treatment (artificial tears) and as active substances delivery vehicles for different ocular pathologies; these preparations are combinations of aqueous mixtures of viscosity-increasing agents (i.e. carboxyvinyl polymers and non-carboxylated cellulose polymers), which result more viscous than the single component - preparations, the patent claims (but does not exemplify) the use of polyethylene glycol or polyvinyl alcohol only in the case of artificial tears.

US-A-5 188 826 teaches topical ophthalmic polymeric suspensions comprising particular lightly crosslinked polymers of acrylic acid, also containing ophthalmic demulcents, vasoconstrictors or combinations thereof.

US-A-3 947 573 teaches an ophthalmic solution for treatment of dry eye syndrome, providing lubricating and cushioning effects for traumatised eyes and as a carrier for ophthalmic medicaments, comprising an aqueous solution of an ethylene oxide polymer, a lower polyalkylene glycol, polyvinyl pyrrolidone and an ophthalmic medicament.

Consequently it appears that, excluding the auto-gelling preparations, the viscosity increase can be desirable in artificial tears (dry eye syndrome treatment), but not in order to increase bioavailability of the active substance (and therefore the therapeutic efficacy of a preparation), due to the poor patient compliance towards vehicles with higher viscosity, and to the easier elimination of the drug by blinking or lacrimal drainage in the case of low viscosity vehicles.

Moreover, the higher costs of manufacturing are a negative element for the competition with conventional eye drops.

### DISCLOSURE OF THE INVENTION.

It has been found that ophthalmic compositions in the form of aqueous gels, characterised by the presence of at least 3 components selected from gelling agents and co-solvents/co-gelling agents, one of which being polyethoxylated derivative of hydrogenated castor oil are particularly effective and well tolerated.

Particularly, the compositions of the invention provide, under similar conditions, the same level of reduction of clinical symptoms, or of cure, obtained by conventional eye drops, even using:
- reduced concentrations of active principles;
- 50% to 70% reduced posology (for example from 4-6 adm./day to 2 adm./day).

These favourable effects cannot be explained only according to the theory of the residence time increase, related to a rise in vehicle viscosity.

The compositions of the invention provide the following advantages:
- a uniform distribution of the administered gel, expecially on the conjunctival region of the cornea, which is the most absorbing zone and, at the same time, an important depot for topically applied drugs. (Reddy I.K. et al. In "Ocular Therapeutic and Drug delivery" - I.K.Reddy Ed.-Technomic Publ., 1996,1-23);
- an optimal adhesion of the film to the corneal and bulbar conjunctival structures;
- a particularly high stabilisation of the above mentioned film, suitable to prevent its rupture and consequently its (partial or total) elimination by lacrimal drainage or its displacement in the lower cul-de-sac zone, where absorption for ocular therapeutical aims is reduced.

The physiological combination of the vehicle (therefore of the active principle) and lacrimal film, without change of its capability to adhere to the cornea and bulbar conjunctiva, could be the basis of the above described advantages.

### DETAILED DISCLOSURE OF THE INVENTION

The invention relates to ophthalmic compositions in the form of aqueous gels with a range of viscosity from 400 to 800 cps, containing:
- at least one active principle,
- a gelling agent,
- at least two co-solubilising/co-gelling agents one of which being polyethoxylated derivatives of hydrogenated castor oil.

Examples of active principles that can be adequately formulated according to the invention include: antibiotic/antibacterial agents (gentamicin, tobramycin and similar ones; chloramphenicol, etc.), antimicotics (miconazole, econazole and similar ones), steroidal and non-steroidal anti-inflammatories (budesonide, diclofenac, niflumic acid, betamethasone and others), beta-blocker such as timolol, etc.

Acrylic acid polymers, sodium carboxymethylcellulose, hydroxymethylcellulose, and/or these mixtures are preferably used as gelling agents.

Among the acrylic acid polymers, Carbomer 940 and 980 are preferably used.

The weight percentages of gelling agents in the final formulations are the following:
- acrylic acid polymers: from 0.1 to 0.7%, preferably from 0.2 to 0.3%;
- sodium carboxymethylcellulose: from 1.5 to 3%, preferably from 2 to 2.5%;
- hydroxymethylcellulose: from 0.5 to 3%, preferably from 1 to 1.5%.

The co-solubilising/co-gelling agents, according to the invention, are selected from: polyethylene glycol (molecular weight between 200 and 500 approx., preferably approx. 300: PEG 300), polyethoxylated hydrogenated castor oil derivatives (Cremophor RH®) and polyvinyl alcohol.

This latter is mainly a co-gelling agent; Cremophor RH is a co-solubilising agent, while PEGs can be considered both co-gelling and co-solubilising agents.

The weight percentages of co-gelling/co-solubilising agents on final formulations are the following ones:
- PEG, particularly for PEG 300, from 4 to 10%;
- Cremophor RH: from 0.5 to 20%, preferably from 2 to 10% and, more preferably, from 2 to 6%;
- polyvinyl alcohol: from 0.4% to 0.5%, preferably 0.4%.

Examples of formulations of this invention could include: ii) PEG 300 and Cremophor RH as co-solubilising/co-gelling agent and sodium carboxymethylcellulose as gelling agent; iii) PEG300 and Cremophor RH as co-solubilising/co-gelling agents and hydroxyethylcellulose as gelling agent.

The following examples show the details of this invention:

| COMPOSITION NO. 1A (steroidal anti-inflammatory / antibiotic) (not according to the invention) | |
|---|---|
| *Active principles:* | |
| Betamethasone 21 -phosphate (equivalent to Betamethasone 0.05%) | 0.066 g |
| Chloramphenicol | 0.250 g |

| *Excipients:* | |
|---|---|
| Polyethylene Glycol 300 | 6.500 g |
| Polyvinyl Alcohol | 0.500 g |
| Carbomer * | 0.290 g |
| Sodium Edetate | 0.100 g |
| Sodium Merthiolate | 0.002 g |
| Sodium Hydroxide q.s. to pH 7.0 | |
| Purified water q.s. to | 100.000 g |

| | |
|---|---|
| * Carbopol 980 | |

| COMPOSITION N O: 1B (steroidal anti-inflammatory / antibiotic ) (not according to the invention) | |
|---|---|
| *Active principles:* | |
| Betamethasone 21 -phosphate (equivalent to Betamethasone 0.1%) | 0.132 g |
| Chloramphenicol | 0.250 g |

| *Excipients:* | |
|---|---|
| Polyethylene Glycol 300 | 6.500 g |
| Polyvinyl Alcohol | 0.500 g |
| Carbomer * | 0.290 g |
| Sodium Edetate | 0.100 g |
| Sodium Merthiolate | 0.002 g |
| Sodium Hydroxide q.s. to pH 7.0 | |
| Purified water q.s. to | 100.000 g |

| | |
|---|---|
| * Carbopol 980 | |

| COMPOSITION NO. 1C (steroidal anti-inflammatory / antibiotic ) | |
|---|---|
| *Active principles:* | |
| Betamethasone 21-phosphate (equivalent to Betamethasone 0.05%) | 0.066 g |
| Chloramphenicol | 0.250 g |

| *Excipients:* | |
|---|---|
| Polyethylene Glycol 300 | 6.500 g |
| Polyvinyl Alcohol | 0.400 g |
| Polyxil 40 hydrogenated castor oil ¹⁾ | 0.100 g |
| Carbomer ²⁾ | 0.290 g |
| Sodium Edetate | 0.100 g |
| Sodium Merthiolate | 0.002 g |
| Sodium Hydroxide q.s. to pH 7.0 | |
| Purified water q.s. to | 100.000 g |

| | |
|---|---|
| ¹⁾ Cremophor RH40 | |
| ²⁾ Carbopol 980 | |

| COMPOSITION NO. 1D (steroidal anti-inflammatory / antibiotic ) | |
|---|---|
| *Active principles:* | |
| Betamethasone 21-phosphate (equivalent to Betamethasone 0.1%) | 0.132 g |
| Chloramphenicol | 0.250 g |

| *Excipients:* | |
|---|---|
| Polyethylene Glycol 300 | 6.500 g |
| Polyvinyl Alcohol | 0.400 g |
| Polyxil 40 hydrogenated castor oil ¹⁾ | 0.100 g |
| Carbomer ²⁾ | 0.290 g |
| Sodium Edetate | 0.100 g |
| Sodium Merthiolate | 0.002 g |
| Sodium Hydroxide q.s. to pH 7.0 | |
| Purified water q.s. to | 100.000 g |

| | |
|---|---|
| ¹⁾ Cremophor RH40 | |
| ²⁾ Carbopol 980 | |

| COMPOSITION NO. 2A (non steroidal anti-inflammatory) (not according to the invention) | |
|---|---|
| *Active principle:* | |
| Diclofenac Sodium | 0.050 g |

| *Excipients:* | |
|---|---|
| Polyethylene Glycol 300 | 6.500 g |
| Polyvinyl Alcohol | 0.500 g |
| Carbomer * | 0.290 g |
| Sodium Edetate | 0.100 g |
| Sodium Merthiolate | 0.002 g |
| Sodium Hydroxide q.s. to pH 7.0 | |
| Purified water q.s. to | 100.000 g |

| | |
|---|---|
| * Carbopol 980 | |

| COMPOSITION NO. 2B (non steroidal anti-inflammatory) (not according to the invention) | |
|---|---|
| *Active principle:* | |
| Diclofenac Sodium | 0.100 g |

| *Excipients:* | |
|---|---|
| Polyethylene Glycol 300 | 6.500 g |
| Polyvinyl Alcohol | 0.500 g |
| Carbomer * | 0.290 g |
| Sodium Edetate | 0.100 g |
| Sodium Merthiolate | 0.002 g |
| Sodium Hydroxide q.s. to pH 7.0 | |
| Purified water q.s. to | 100.000 g |

| | |
|---|---|
| * Carbopol 980 | |

| COMPOSITION NO. 2C (non steroidal anti-inflammatory) | |
|---|---|
| *Active principle:* | |
| Diclofenac Sodium | 0.050 g |

| *Excipients:* | |
|---|---|
| Polyethylene Glycol 300 | 6.400 g |
| Polyxil 40 hydrogenated castor oil ¹⁾ | 0.100 g |
| Polyvinyl Alcohol | 0.500 g |
| Carbomer ²⁾ | 0.290 g |
| Sodium Edetate | 0.100 g |
| Sodium Merthiolate | 0.002 g |
| Sodium Hydroxide q.s. to pH 7.0 | |
| Purified water q.s. to | 100.000 g |

| | |
|---|---|
| ¹⁾ Cremophor RH40 | |
| ²⁾ Carbopol 980 | |

| COMPOSITION NO. 2D (non steroidal anti-inflammatory) | |
|---|---|
| *Active principle*: | |
| Diclofenac Sodium | 0.100 g |

| *Excipients:* | |
|---|---|
| Polyethylene Glycol 300 | 6.400 g |
| Polyxil 40 hydrogenated castor oil ¹⁾ | 0.100 g |
| Polyvinyl Alcohol | 0.500 g |
| Carbomer ²⁾ | 0.290 g |
| Sodium Edetate | 0.100 g |
| Sodium Merthiolate | 0.002 g |
| Sodium Hydroxide q.s. to pH 7.0 | |
| Purified water q.s. to | 100.000 g |

| | |
|---|---|
| ¹⁾ Cremophor RH40 | |
| ²⁾ Carbopol 980 | |

| COMPOSITION NO. 3A (non steroidal anti-inflammatory ) | |
|---|---|
| *Active principle:* | |
| Niflumic Acid | 0.500 g |

| *Excipients:* | |
|---|---|
| polyethylene Glycol 300 | 6.500 g |
| Sodium Carboxymethylcellulose | 2.500 g |
| Sodium Chloride | 0.570 g |
| Polyxil 40 hydrogenated castor oil* | 0.500 g |
| Sodium Merthiolate | 0.002 g |
| Sodium Hydroxide q.s. to pH 7.0 | |
| Purified water q.s. | 100.000 g |

| | |
|---|---|
| * Cremophor RH-40 | |

| COMPOSITION NO. 3B (non steroidal anti-inflammatory) | |
|---|---|
| *Active principle:* | |
| Niflumic Acid | 1.000 g |

| *Excipients:* | |
|---|---|
| Polyethylene Glycol 300 | 6.500 g |
| Sodium Carboxymethylcellulose | 2.500 g |
| Sodium Chloride | 0.570 g |
| Polyxil 40 hydrogenated castor oil * | 0.500 g |
| Sodium Merthiolate | 0.002 g |
| Sodium Hydroxide q.s. to pH 7.0 | |
| Purified water q.s. to | 100.000 g |

| | |
|---|---|
| * Cremophor RH-40 | |

| COMPOSITION NO. 4A (Antifungal) | |
|---|---|
| *Active principle:* | |
| Miconazole | 0.300 g |

| *Excipients:* | |
|---|---|
| Polyethylene Glycol 300 | 10.000 g |
| Polyoxil 40 hydrogenated castor oil * | 6.000 g |
| Sodium Carboxymethylcellulose | 2.500 g |
| Gluconic Acid | 0.150 g |
| Cetrimide | 0.010 g |
| Sodium Hydroxide q.s. to pH 7.0 | |
| Purified water q.s. to | 100.000 g |

| | |
|---|---|
| * Cremophor RH-40 | |

| COMPOSITION NO. 4B (Antifungal) | |
|---|---|
| *Active principle:* | |
| Miconazole | 0.500 g |

| *Excipients:* | |
|---|---|
| Polyethylene Glycol 300 | 10.000 g |
| Polyoxil 40 hydrogenated castor oil* | 6.000 g |
| Sodium Carboxymethylcellulose | 2.500 g |
| Gluconic Acid | 0.250 g |
| Cetrimide | 0.010 g |
| Sodium Hydroxide q.s. to pH 7.0 | |
| Purified water q.s. to | 100.000 g |

| | |
|---|---|
| * Cremophor RH40 | |

| COMPOSITION NO. 5A (Anti-glaucoma) | |
|---|---|
| *Active principle:* | |
| Timolol maleate (equivalent to Timolol 0.25 %) | 0.342 g |

| *Excipients:* | |
|---|---|
| Polyethylene Glycol 300 | 3.000 g |
| Sodium Carboxymethylcellulose | 2.000 g |
| Polyoxil 40 hydrogenated castor oil * | 2.000 g |
| Sodium Chloride | 0.165 g |
| Sodium Edetate | 0.100 g |
| Benzalkonium Chloride | 0.010 g |
| Sodium Hydroxide q.s. to pH 7.0 | |
| Purified water q.s. to | 100.000 g |

| | |
|---|---|
| * Cremophor RH40 | |

| COMPOSITION NO. 5B (Anti-glaucoma) | |
|---|---|
| *Active principle:* | |
| Timolol maleate (equivalent to Timolol 0.50 %) | 0.684 g |

| *Excipients:* | |
|---|---|
| Polyethylene Glycol 300 | 3.000 g |
| Sodium Carboxymethylcellulose | 2.000 g |
| Polyoxil 40 hydrogenated castor oil * | 2.000 g |
| Sodium Chloride | 0.165 g |
| Sodium Edetate | 0.100 g |
| Benzalkonium Chloride | 0.010 g |
| Sodium Hydroxide q.s. to pH 7.0 | |
| Purified water q.s. to | 100.000 g |

| | |
|---|---|
| * Cremophor RH40 | |

| COMPOSITION NO. 6 (Antibiotic / non steroidal anti-inflammatory) | |
|---|---|
| *Active principles:* | |
| Diclofenac Sodium | 0.100 g |
| Tobramycin | 0.300 g |

| *Excipients:* | |
|---|---|
| Polyethylene Glycol 300 | 4.000 g |
| Polyoxil 40 hydrogenated castor oil* | 2.000 g |
| Cellulose Hydroxyethyl | 1.500 g |
| Sodium Chloride | 0.800 g |
| Cetrimide | 0.010 g |
| Hydrogen Chloride q.s. to pH 7.0 | |
| Purified water q.s. to | 100.000 g |

| | |
|---|---|
| * Cremophor RH40 | |

| COMPOSITION NO. 7A (Antibiotic) | |
|---|---|
| *Active principle:* | |
| Gentamicin Sulphate (equivalent to Gentamicin 0.15%) | 0.250 g |

| *Excipients:* | |
|---|---|
| Polyethylene Glycol 300 | 4.000 g |
| Cellulose Hydroxyethyl | 1.500 g |
| Sodium Phosphate Dibasic | 0.630 g |
| Polyoxil 40 hydrogenated castor oil * | 0.500 g |
| Sodium Chloride | 0.500 g |
| Sodium Phosphate Monobasic | 0.170 g |
| Sodium Edetate | 0.100 g |
| Sodium Metabisulfite | 0.100 g |
| Benzalkonium Chloride | 0.010 g |
| Sodium Hydroxide q.s. to pH 7.0 | |
| Purified water q.s. to | 100.000 g |

| | |
|---|---|
| * Cremophor RH40 | |

| COMPOSITION NO. 7B (Antibiotic) | |
|---|---|
| *Active principle:* | |
| Gentamicin Sulphate (equivalent to Gentamicin 0.3%) | 0.500 g |

| *Excipients:* | |
|---|---|
| Polyethylene Glycol 300 | 4.000 g |
| Cellulose Hydroxyethyl | 1.500 g |
| Sodium Phosphate Dibasic | 0.630 g |
| Polyoxil 40 hydrogenated castor oil * | 0.500 g |
| Sodium Chloride | 0.500 g |
| Sodium Phosphate Monobasic | 0.170 g |
| Sodium Edetate | 0.100 g |
| Sodium Metabisulfite | 0.100 g |
| Benzalkonium Chloride | 0.010 g |
| Sodium Hydroxide q.s. to pH 7.0 | |
| Purified water q.s. to | 100.000 g |

| | |
|---|---|
| * Cremophor RH40 | |

| COMPOSITION NO. 8A (Anti-glaucoma) | |
|---|---|
| *Active principle:* | |
| Timolol maleate (equivalent to Timolol 0.25 %) | 0.342 g |

| *Excipients:* | |
|---|---|
| Polyethylene Glycol 300 | 3.000 g |
| Polyoxil 40 hydrogenated castor oil * | 2.000 g |
| Cellulose Hydroxyethyl | 1.200 g |
| Sodium Chloride | 0.280 g |
| Sodium Edetate | 0.100 g |
| Benzalkonium Chloride | 0.010 g |
| Sodium Hydroxide q.s. to pH 7.0 | |
| Purified water q.s. to | 100.000 g |

| | |
|---|---|
| * Cremophor RH40 | |

| COMPOSITION NO. 8B (Anti-glaucoma) | |
|---|---|
| *Active principle:* | |
| Timolol maleate (equivalent to Timolol 0.50 %) | 0.684 g |

| *Excipients:* | |
|---|---|
| Polyethylene Glycol 300 | 3.000 g |
| Polyoxil 40 hydrogenated castor oil * | 2.000 g |
| Cellulose Hydroxyethyl | 1.200 g |
| Sodium Chloride | 0.280 g |
| Sodium Edetate | 0.100 g |
| Benzalkonium Chloride | 0.010 g |
| Sodium Hydroxide q.s. to pH 7.0 | |
| Purified water q.s. to | 100.000 g |

| | |
|---|---|
| * Cremophor RH40 | |

## Claims

1. Ophthalmic compositions in the form of aqueous gel with a viscosity range between 400 and 800 mPa.s (cps) comprising:
- one active principle;
- a gelling agent and at least two co-solubilising/co-gelling agents, one of which being polyethoxylated derivatives of hydrogenated castor oil.

2. Compositions as claimed in claim 1, wherein the gelling agent is selected from acrylic acid polymers, sodium carboxymethylcellulose, hydroxyethylcellulose.

3. Compositions as claimed in claim 1 or 2, wherein the co-solubilising/co-gelling agent is selected from a polyethylene glycol with molecular weight ranging from 200 to 500, polyethoxylated derivatives of hydrogenated castor oil, polyvinyl alcohol.

4. Compositions as claimed in any one of the above claims wherein the acrylic acid polymer is Carbomer 980.

5. Compositions as claimed in any one of the above claims wherein one of the two co-soiubiiising/co-gelling agents is PEG300.

6. Compositions as claimed in claim 5 wherein the other co-solubilising/co-gelling is polyvinyl alcohol.

7. Compositions as claimed in any one of the above claims, containing an ophthalmically useful active principle selected from antibiotics, antibacterials, antimicotics, steroidal or non steroidal anti-inflammatories, beta-blockers.

## Patentansprüche

1. Ophthalmische Zusammensetzungen in Form eines wasserhaltigen Gels mit einem Viskositätsbereich zwischen 400 und 800 mPa.s (cps), umfassend:
- einen Wirkstoff;
- ein Geliermittel und mindestens zwei Co-Solubilisierungs-/Co-Geliermittel, wobei eines davon aus polyethoxylierten Derivaten von hydriertem Castoröl ausgewählt ist.

2. Zusammensetzungen nach Anspruch 1, wobei das Geliermittel ausgewählt ist aus Acrylsäurepolymeren, Natriumcarboxymethylcellulose, Hydroxyethylcellulose.

3. Zusammensetzungen nach Anspruch 1 oder 2, wobei das Co-Solubilisierungs-/Co-Geliermittel ausgewählt ist aus einem Polyethylenglykol mit einem Molekulargewicht von 200 bis 500, polyethoxylierten Derivaten von hydriertem Castoröl, Polyvinylalkohol.

4. Zusammensetzungen nach einem der vorherigen Ansprüche, wobei das Acrylsäurepolymer Carbomer 980 ist.

5. Zusammensetzungen nach einem der vorherigen Ansprüche, wobei eines der zwei Co-Solubilisierungs-/Co-Geliermittel PEG300 ist.

6. Zusammensetzungen nach Anspruch 5, wobei das andere Co-Solubilisierungs-/Co-Geliermittel Polyvinylalkohol ist.

7. Zusammensetzungen nach einem der vorherigen Ansprüche, die einen ophthalmisch verwendbaren Wirkstoff enthalten, ausgewählt aus Antibiotika, Bakteriziden, Antimykotika, steroidalen oder nicht-steroidalen Entzündungshemmern, beta-Blockern.

## Revendications

1. Compositions ophtalmiques sous forme de gels aqueux avec une plage de viscosité comprise entre 400 et 800 mPa.s (cps) comprenant:
- un principe actif ;
- un agent de gélification et au moins deux agents de co-solubilisation/co-gélification, l'un des deux agents étant un dérivé polyethoxylé de l'huile de ricin hydrogenée.

2. Compositions telles que revendiquées dans la revendication 1, dans lesquelles l'agent de gélification est choisi parmi les polymères d'acide acrylique, la carboxyméthylcellulose sodique, l'hydroxyéthylcellulose.

3. Compositions telles que revendiquées dans la revendication 1 ou 2, dans lesquelles l'agent de co-solubilisation/co-gélification est choisi parmi le polyéthylène glycol ayant un poids moléculaire se situant dans la plage de 200 à 500, des dérivés polyéthoxylés de l'huile de ricin hydrogénée et l'alcool polyvinylique.

4. Compositions telles que revendiquées dans l'une quelconque des revendications ci-dessus, dans lesquelles le polymère d'acide acrylique est le Carbomer 980.

5. Compositions telles que revendiquées dans l'une quelconque des revendications ci-dessus, dans lesquelles l'un des deux agents de co-solubilisation/co-gélification est le PEG300.

6. Compositions telles que revendiquées dans la revendication 5, dans lesquelles l'autre agent de co-solubilisation/co-gélification est l'alcool polyvénylique.

7. Compositions telles que revendiquées dans l'une quelconque des revendications ci-dessus, contenant un principe actif utile en ophtalmologie choisi parmi les antibiotiques, les antibactériens, les anti-micotiques, les anti-inflammatoires stéroïdiens ou non stéroïdiens, les bêta-bloquants.
